# EUROPEAN PATENT SPECIFICATION

(11) **EP 2 731 441 B1**
(45) Date of publication and mention of the grant of the patent: **10.02.2016**
(21) Application number: 12751363.8
(22) Date of filing: 09.07.2012
(51) Int. Cl.: A01N 63/04, C12R 1/645

(54) **NEW YEAST STRAINS AND USE THEREOF FOR THE CONTROL OF PHYTOPATHOGENIC FUNGI**
NEUE HEFESTÄMME UND IHRE VERWENDUNG ZUR BEKÄMPFUNG VON PHYTOPATHOGENEN PILZEN
NOUVELLES SOUCHES DE LEVURES ET LEUR UTILISATION EN VUE DE LA LUTTE CONTRE LES CHAMPIGNONS PHYTOPATHOGÈNES

(30) Priority: 11.07.2011 IT MI20111292
(43) Date of publication of application: 21.05.2014
(73) Proprietor: Universita' Degli Studi Del Molise, 86100 Campobasso (IT)
(72) Inventor: DE CURTIS, Filippo, I-86035 Larino (CB) (IT); CASTORIA, Raffaello, I-00143 Roma (IT); LIMA, Giuseppe, I-86010 Ferrazzano (CB) (IT); DE CICCO, Vincenzo, I-70121 Bari (BA) (IT)
(74) Representative: Finetti, Claudia
(86) International application number: PCT/IB2012/053507
(87) International publication number: WO 2013/008173

(56) References cited:
- WO-A2-2008/114304
- GIUSEPPE LIMA ET AL: "Integrated control of blue mould using new fungicides and biocontrol yeasts lowers levels of fungicide residues and patulin contamination in apples", POSTHARVEST BIOLOGY AND TECHNOLOGY, ELSEVIER, NL, vol. 60, no. 2, 20 December 2010 (2010-12-20), pages 164-172, XP028171762, ISSN: 0925-5214, DOI: 10.1016/J.POSTHARVBIO.2010.12.010 [retrieved on 2010-12-24]
- TOLAINI V ET AL: "Lentinula edodes enhances the biocontrol activity of Cryptococcus laurentii against Penicillium expansum contamination and patulin production in apple fruits", INTERNATIONAL JOURNAL OF FOOD MICROBIOLOGY, ELSEVIER SCIENCE PUBLISHERS, AMSTERDAM, NL, vol. 138, no. 3, 15 April 2010 (2010-04-15), pages 243-249, XP026979159, ISSN: 0168-1605 [retrieved on 2010-02-14]
- KOMAGATA ET AL: "Value of chemosystematic data for predicting anamorph-teleomorph relationships between the genera Rhodotorula and Rhodosporidium", FEMS MICROBIOLOGY LETTERS, NO LONGER PUBLISHED BY ELSEVIER, vol. 100, no. 1-3, 15 December 1992 (1992-12-15), pages 503-508, XP026922682, ISSN: 0378-1097 [retrieved on 1992-12-15]

## Description

The present invention relates to new yeast strains and their use, alone or in a synergistic mixture, for the biological control of phytopathogenic fungi, also mycotoxigenic, of agricultural crops of considerable economical interest, and the relative fungicidal compositions.

The use of yeasts belonging to the species *Aureobasidium, Rhodotorula, Cryptococcus, Metschnikowia, Candida,* etc., for the control of phytopathogenic fungi, in particular those responsible for fruit rot after harvesting, is already known and described, for example, in patent applications WO96/25039 (*Rhodotorula, Cryptococcus*), US5244680 (*Cryptococcus*), US5843434 (*Candida*), WO02/072777 (*Metschnikowia*), WO2008/114304 (*Aureobasidium, Rhodotorula, Cryptococcus*), WO2009/040862 (*Metschinikowia*), as well as in the bibliographical references quoted in said documents.

WO2008/114304 discloses, among other strains, a strain of *Rhodotorula glutinis* coded LS11, and a strain of *Cryptococcus laurentii* coded LS28, which can both be used for the control of phytopathogenic fungi, among which also various producers of mycotoxins (mycotoxigenic fungi). These two yeasts, however, when applied individually or in combination in antifungal field treatment, before harvesting, as such or in suitable experimental formulations, under environmental conditions favourable for the onset and development of the disease, have not always provided satisfactory results, comparable to those obtained with synthetic products formulates. These results, not always positive, can be attributed to the following factors:
a) survival rate of the two microorganisms on the vegetable surfaces, not always sufficient for effectively opposing the pathogen action;
b) poor compatibility between the two strains when applied in combination;
c) limited aptitude of the two strains for the development of formulations with a sufficiently long shelf-life.

The Applicant has now isolated two new yeast strains, one belonging to the species *Rhodosporidium kratochvilovae* and the other to the species *Cryptococcus laurentii.* The yeast strains *Rhodosporidium kratochvilovae* and *Cryptococcus laurentii* were both filed for patent purposes, according to the procedures established by the Budapest Treaty, at the Industrial Yeasts Collection DBVPG of the Applied Biology Department, University of Perugia, Borgo XX Giugno 74, I-06121 Perugia (Italy), on 20/12/2009 and 9/5/2011, with the filing numbers 26P and 31P respectively.

These two new yeast strains, when tested against different fungal pathogens on different crops, not only prove to be highly compatible and synergistic with each other, but are also surprisingly much more effective with respect to the strain *Rhodotorula glutinis* coded LS11 and the strain *Cryptococcus laurentii* coded LS28, previously described in WO2008/114304.

The new strain of R. *kratochvilovae* (DBVPG 26P) was isolated from olive drupes, whereas the new strain of *C. laurentii* (DBVPG 31P) was isolated from the fruit of apple trees.

The two new yeast strains were obtained by means of selective isolation in an artificial agarized medium (Nutrient Yeast Extract Dextrose Agar: NYDA) using the technique described by Wilson [Wilson et al., Scientia Hort. (1993) 53: pages 183-189]. In short, this technique envisages the application of washing water obtained from fruit and/or leaves directly *in vivo*, in artificial lesions of apples subsequently inoculated with spores of fungal pathogens. With the lesions which, in the subsequent days, do not develop any evident infections, a re-insulation and purification is effected in culture of microorganisms with a probable antagonistic activity and potentially responsible for the non-development of the disease. The potentially antagonistic microorganisms selected are then further tested and characterized for antifungal activity on different species of plants of agricultural interest and against different pathogens. The strains held as being potentially antagonistic were typified from a genetic-molecular point of view.

The two new strains DBVPG 26P and DBVPG 31P, used either alone or in a synergistic mixture, as such or preferably in the form of suitable phytosanitary compositions, have proved to be much more effective than the strains described in the known art for the control of phytopathogenic fungi (also mycotoxigenic) of important agricultural crops, not only in post-harvest applications but also in traditional fungicidal field treatment during the development of the crops and up to before harvesting.

Furthermore, the two new yeast strains, alone or mixed, prove to be much more suitable, with respect to the previous strains, for obtaining new preparations which, in addition to guaranteeing higher levels of efficacy with respect to fungal pathogens, also mycotoxigenic, have an unusual and longer shelf-life.

On the basis of the not always satisfactory survival limits on vegetable surfaces and antifungal activity shown by the yeast strains previously indicated in the known art, the new strains DBVPG 26P and DBVPG 31P survive, and therefore persist, more effectively and for a longer period on undamaged or damaged vegetable surfaces, and exert a higher antifungal activity.

In addition, due to:
i) the high compatibility and strong synergism of the two new strains when mixed;
ii) the high compatibility of the two strains when used in combination with different adjuvants;
iii) the high compatibility of the two strains when used in combination with fungicides commonly used in biological or integrated agriculture;
iv) their more prolonged shelf-life when formulated alone or mixed with each other;
the two new yeast strains are capable of providing much higher protection levels against open-field fungal attacks and during post-harvest on different and important agricultural crops, comparable with, and sometimes better than, those that can be obtained by using the traditional chemical protection.

More specifically, the synergism shown by the two new strains when applied in a mixture with each other, is due to their different and complementary biological characteristics. The two new strains, in fact, have different mechanisms of action and environmental adaptation, and colonize vegetables differently.

Furthermore, the new strains DBVPG 26P and DBVPG 31P produce high enzymatic activities, capable of preventing, through the lysis of the polymers of the fungal cell walls, the development of phytopathogenic fungi, with a higher activity on the part of the strain DBVPG 31P.

The latter also has a surprising resistance to oxidative stress generated by substances produced by the damaged vegetable tissue which represents the centre of infection for many phytopathogenic fungi, thus having a higher colonization capacity of said damaged tissue.

Even if the two strains are both capable of developing in different environmental conditions, the strain DBVPG 26P of *R. kratochvilovae*, with respect to the strain DBVPG 31P of *C. laurentii*, colonizes undamaged vegetable surfaces better, has a better development at low temperatures, showing a higher persistence also on cold-stored fruit and vegetables , and has a surprising capacity of degrading mycotoxin patulin, much higher than other examples indicated in the known art. The strain DBVPG 26P, moreover, represents the first example of *Rhodosporidium kratochvilovae* which can be used as biopesticide.

As already mentioned, these two new yeast strains have proved to be surprisingly more effective than the strains known in the art for the control of phytopathogenic fungi which attack the main agricultural crops.

An object of the present invention therefore relates to:
- the yeast strain *Rhodosporidium kratochvilovae* deposited on 20/12/2009 at the Industrial Yeasts Collection DBVPG, of the Dipartimento di Biologia Applicata (Department of Applied Biology), University of Perugia, Borgo XX Giugno, 74, I-06121 Perugia (Italy) with the deposit number 26P;
- the yeast strain *Cryptococcus laurentii* deposited on 09/05/2011 at the Industrial Yeasts Collection DBVPG, of the Dipartimento di Biologia Applicata (Department of Applied Biology), University of Perugia, Borgo XX Giugno, 74, I-06121 Perugia (Italy) with the deposit number 31P.

A further object of the present invention relates to a mixture of the two yeast strains *Rhodosporidium kratochvilovae* DBVPG 26P and *Cryptococcus laurentii* DBVPG 31P. The application of the two microorganisms in a mixture, has in fact proved to be particularly advantageous for the purposes of controlling phytopathogenic fungi, also mycotoxigenic, of agricultural crops, as an extremely high synergistic effect has been found, which is exerted in a surprisingly high antifungal efficacy, higher than that expected on the basis of the efficacy of the two yeasts used separately and calculated using the formulae of Colby [Colby, "Weeds", 15 (1967), pages 20-22] and Abbott [Levy et al., "EPPO Bull." 16 (1986), 8, pages 651-657].

As mentioned above, for practical use it is preferable to utilize the two microorganisms, alone or mixed with each other, in the form of suitable phytosanitary fungicidal compositions, which represent a further aspect of the present invention.

They can be obtained by the drying or lyophilization of the biomass deriving from the fermentation of the two yeast strains, in turn obtained by filtration, sedimentation or centrifugation of the respective fermentation broths.

The present invention consequently further relates to a fungicidal composition characterized in that it comprises one of the two yeast strains according to the present invention or a mixture thereof, as active principle, together with other phytopharmacologically acceptable adjuvants and/or excipients.

In a preferred embodiment of the invention, said fungicidal composition comprises a synergic mixture obtained by the association of *Rhodosporidium kratochvilovae*, coded DBVPG 26P, with *Cryptococcus laurentii*, coded DBVPG 31P, in proportions varying from 80/20 (4:1) to 20/80 (1:4) in terms of Colony Forming Units (CFU). The most suitable percentage ratio between the two strains in the mixture depends on the pathogen to be controlled, the ecosystem in which the mixture has to be applied (plant species of the crop to be treated), the environment in which the mixture is to be used (open field or cold-storage), in addition to the phenological phase of the crop to be treated.
In the above compositions, the concentration of *Rhodosporidium kratochvilovae* DBVPG 26P, and/or of *Cryptococcus laurentii* coded DBVPG 31P, can vary from 5x10⁶ CFU/ml (Colony Forming Units = number of cells per 1 millilitre of the mixture suspension) to 1x10⁸ CFU/ml; it generally ranges from 5x10⁷ UFC/ml to 1x10⁸ CFU/ml.

According to a further embodiment, said fungicidal composition is characterized in that it includes at least one further active ingredient selected from fungicides, phytoregulators, antibiotics, herbicides, acaricides, insecticides, fertilizers, food additives, antioxidants and/or mixtures thereof.

Examples of fungicides which can be included in the compositions, object of the present invention, are: acibenzolar, ametoctradin, amisulbrom, ampropylfos, anilazine, azaconazole, azoxystrobin, benalaxyl, benalaxyl-M, benomyl, benthiavalicarb, bitertanol, bixafen, blasticidin-S, boscalid, bromuconazole, bupirimate, buthiobate, captafol, captan, carbendazim, carboxin, carpropamid, chinomethionat, chloroneb, chlorothalonil, chlozolinate, cyazofamid, cyflufenamid, cymoxanil, cyproconazole, cyprodinil, debacarb, dichlofluanid, dichlone, diclobutrazol, diclomezine, dicloran, diclocymet, diethofencarb, difenoconazole, diflumetorim, dimethirimol, dimethomorph, dimoxystrobin, diniconazole, dinocap, dipyrithione, ditalimfos, dithianon, dodemorph, dodine, edifenphos, epoxiconazole, etaconazole, ethaboxam, ethirimol, ethoxyquin, etridiazole, famoxadone, fenamidone, fenaminosulf, fenapanil, fenarimol, fenbuconazole, fenfuram, fenhexamid, fenoxanil, fenpiclonil, fenpropidin, fenpropimorph, fentin, ferbam, ferimzone, fluazinam, fludioxonil, flumetover, flumorph, fluopicolide, fluopyram, fluoroimide, fluotrimazole, fluoxastrobin, fluquinconazole, flusilazole, flusulfamide, flutianil, flutolanil, flutriafol, folpet, fosetyl-aluminium, fuberidazole, furalaxyl, furametpyr, furconazole, furconazole-cis, guazatine, hexaconazole, hymexazol, idrossichinolina solfato, imazalil, imibenconazole, iminoctadine, ipconazole, iprobenfos, iprodione, isoprothiolane, iprovalicarb, isopyrazam, isotianil, kasugamycin, kresoxim-methyl, mancopper, mancozeb, mandipropamid, maneb, mebenil, mepanipyrim, mepronil, meptyl dinocap, metalaxyl, metalaxyl-M, metconazole, methfuroxam, metiram, metominostrobin, metrafenone, metsulfovax, myclobutanil, natamycin, nicobifen, nitrothalisopropyl, nuarimol, ofurace, orysastrobin, oxadixyl, oxpoconazole, oxycarboxin, pefurazoate, penconazole, pencycuron, penflufen, pentachlorofenol and salts thereof, penthiopyrad, phthalide, picoxystrobin, piperalin, Bordeaux mixture, polyoxins, probenazole, prochloraz, procymidone, propamocarb, propiconazole, propineb, proquinazid, prothiocarb, prothioconazole, pyracarbolid, pyraclostrobin, pyrametostrobin, pyraoxystrobin, pyrazophos, pyribencarb, pyrifenox, pyrimethanil, pyroquilon, pyroxyfur, quinacetol, quinazamid, quinconazole, quinoxyfen, quintozene, rabenzazole, copper hydroxide, copper oxichloride, cuprous oxide, copper sulfate, sedaxane, silthiofam, simeconazole, spiroxamine, streptomycin, tebuconazole, tetraconazole, thiabendazole, thiadifluor, thicyofen, thifluzamide, thiophanate, thiophanate-methyl, thiram, tiadinil, tioxymid, tolclofos-methyl, tolylfluanid, triadimefon, triadimenol, triarimol, triazbutil, triazoxide, tricyclazole, tridemorf, trifloxystrobin, triflumizole, triforine, triticonazole, uniconazole, uniconazole-P, validamycin, valifenalate, vinclozolin, zineb, ziram, sulfur, zoxamide.

The fungicidal compositions according to the present invention can be in the form of powders or aqueous solutions which, in turn, are diluted with suitable quantities of water so as to obtain dispersions which are partially or totally insoluble in water, or more diluted solutions.

As already mentioned, the above compositions can be effectively used in fungicidal treatment, both in the open field, up until harvesting, and also in post-harvesting.

In particular, the compositions comprising the yeast strains according to the invention, have proved to be extremely suitable for the control of *Plasmopara viticola* on vines, *Phytophthora infestans* and *Botrytis cinerea* on tomatoes, *Puccinia recondita, Erysiphe(=Blumeria) graminis, Helminthosporium teres, Septoria nodorum* and *Fusarium* spp. on cereals in the control of *Phakopsora pachyrhizi* on soya, for controlling *Uromyces appendiculatus* on beans, for controlling *Venturia inaequalis* on apple trees, in the control of *Sphaeroteca fuliginea* on cucumbers.

A further aspect of the present invention therefore relates to the use of the single strains or a mixture thereof or fungicidal compositions comprising the strain *Rhodosporidium kratochvilovae* coded DBVPG 26P, and/or the strain *Cryptococcus laurentii* coded DBVPG 31P, for the control of phytopathogenic fungi, also mycotoxigenic, of agricultural crops.

The greater efficacy obtained with the mixture of the two strains (use of the strains in a combination), as already specified, derives from the complementary nature of:
i) the mechanisms of action of the two strains;
ii) the different capacities of overcoming the unfavourable weather conditions (high and low temperatures with low and high humidity levels);
iii) the capacity of colonizing undamaged and damaged vegetable surfaces.

Examples of phytopatogenic fungi which can be effectively controlled with the two strains described, used alone or in a synergistic mixture, are those belonging to the group of *Basidiomycetes, Ascomycetes, Deuteromycetes* or imperfect fungi, *Oomycetes: Puccinia* spp., *Ustilago* spp., *Tilletia* spp., *Uromyces* spp., *Claviceps purpurea, Phakopsora* spp., *Rhizoctonia* spp., *Sclerotium rolfsii, Sclerotium cepivorum, Erysiphe* spp., *Sphaerotheca* spp., *Podosphaera* spp., *Uncinula* spp., *Helminthosporium* spp., *Rhynchosporium* spp., *Pyrenophora* spp., *Aspergillus* spp., *Botrytis* spp., *Monilinia* spp., *Sclerotinia* spp., *Rhizopus* spp., *Mucor* spp., *Septoria* spp. *(Mycosphaerella* spp.), *Phaeosphaeria* spp., *Venturia* spp., *Alternaria* spp., *Fusarium* spp., *Cercospora* spp., *Cladosporium* spp., *Phoma* spp., *Cercosporella herpotrichoides, Colletotrichum* spp., *Pyrenochaeta lycopersici, Pyricularia oryzae, Sclerotium* spp., *Ascochyta* spp., *Albugo* spp., *Phytophthora* spp., *Pythium* spp., *Plasmopara viticola, Thielaviopsis basicola, Diaporthe* spp, *Peronospora* spp., *Pseudoperonospora cubensis, Bremia lactucae, Penicillium* spp., *Cladosposium* spp., *Stemphylium* spp., *Leveillula taurica.*

The main crops which can be protected with the microorganisms of the present invention comprise cereals (wheat, barley, rye, oat, rice, corn, sorghum, etc.), fruit trees (olive trees, apple trees, pear trees, apricot trees, nashi trees, plum trees, peach trees, almond trees, cherry trees, kaki trees, banana trees, grapevines, strawberries, raspberries, blackberries, etc.) citrus trees (orange trees, lemon tree, tangerine trees, clementine trees, grapefruit trees, etc.) legumes (beans, peas, lentils, soybeans (etc.) horticultural crops (spinach, lettuce, asparagus, artichokes, cabbage, carrots, onions, garlic, tomatoes, potatoes, aubergines, peppers, fennels etc.) cucurbits (pumpkins, marrows, cucumbers, melons, watermelons etc.), oleaginous plants (soybeans, sunflowers, rapes, peanuts, castor oil, coconuts, etc.), tobacco, coffee, tea, cacao, sugar beet, sugar cane, cotton.

In particular, the yeast strains according to the invention have proved to be extremely effective in controlling *Plasmopara viticola* on vines, *Phytophthora infestans* and *Botrytis cinerea* on tomatoes, *Puccinia recondita, Erysiphe (=Blumeria) graminis, Helminthosporium teres, Septoria nodorum and Fusarium* spp. on cereals, in controlling *Phakopsora pachyrhizi* on soybeans, in controlling *Uromyces appendiculatus* on beans, in controlling *Venturia inaequalis* on apple trees, in controlling *Sphaeroteca fuliginea* on cucumbers.

In a preferred embodiment, said strains are used in the dried, lyophilized, frozen, liquid state.

The mixture of yeasts or the fungicidal composition comprising the yeasts according to the invention, alone or in combination, is preferably formulated in the form of a dry powder, wettable powder, solution, dispersion or suspension.

A further object of the present invention relates to a method for the control of phytopatogenic fungi, also mycotoxigenic, in agricultural crops which consists in the pre- or post-harvest application of effective dosages of *Rhodosporidium kratochvilovae* coded DBVPG 26P, and/or *Cryptococcus laurentii* coded DBVPG 31P, used as such or formulated in fungicidal compositions as described above.

Examples of phytopatogenic fungi that can be controlled and crops which can be protected with the above compositions, are those indicated above.

The application of the two new yeast strains, as such or in the form of suitable compositions, can be effected on any undamaged or damaged surface of the plant, for example on the leaves, flowers, fruit, stems, buds, branches and roots, or on the seeds before sowing or on the ground in which the plant grows.

The treatments in open field, on the leaves, flowers, fruit, stems and branches, on the roots or on the ground can be effected by distributing the microorganisms as such, or compositions thereof, with usual mechanical means suitable for the distribution of pesticidal products.

The treatments on fruit during the post-harvest phase in cold-storage cells, can be effected by applying the microorganisms or compositions thereof with distribution equipments currently used by experts in the field.

Preferably, the dosage used in applications according to the method for the fungicidal treatment of plants of the present invention, ranges from 1 to 20 kg per hectare of composition containing the yeast strain(s).

This treatment can be of a preventive, curative or eradicating nature.

The quantity of yeast to be applied for obtaining the desired effect can vary in relation to different factors such as: the crop to be protected, the phenological stage of the crop, the type of pathogen/disease, the degree of infection, the climatic conditions, the application method, the selected formulation.

The concentrations of yeast(s) to be used preferably range from 1x10⁶ to 1x10⁸ CFU/ml. The concentration depends on the crop to be protected, the phenological period, the type of pathogen/disease to be controlled, the degree of infection, the climatic conditions, the application method, the selected formulation.

The yeast cell concentration to be utilized is lower, with respect to the known art, when the two new yeasts are used in a mixture, thanks to the complementary nature and synergism deriving from their combination. Furthermore, the cell concentration of the two new yeasts to be utilized in the mixture, is lower with respect to the known art, when they are used in a combination with additives and/or synthetic active substances, thus producing, with the same fungicidal effectiveness, a considerable cost savings in the production of the biomass by fermentation.

The present invention will be now described for illustrative but non-limiting purposes, according to two preferred embodiments, with particular reference to the following examples.

### EXAMPLE 1: Isolation and fermentation of the strain of Rhodosporidium kratochvilovae DBVPG 26P.

The strain *Rhodosporidium kratochvilovae* DBVPG 26P was isolated from olive drupes according to the technique described by Wilson et al., 1993. Samples (50 gr) of olive drupes taken from organically grown olive plants, cv Salegna, were introduced for the purpose into 1000 ml flasks containing 500 ml of steril distilled water on a rotary shaker at 23°C. After 30 minutes, 100 µl of washing water were applied directly to artificial lesions of fruit which were subsequently inoculated with spores of fungal pathogens.

The subsequent days, from the lesions in which there was no development of evident infections, re-isolation and purification in the medium of microorganisms having a probable antagonistic activity and potentially responsible for the non-development of the disease, were performed. The potential antagonistic microorganisms selected were then further tested and characterized for antifungal activity.

In order to obtain the culture suspension of the strain DBVPG 26P of *Rhodosporidium kratochvilovae,* 5 litre flasks containing the culture medium NYDB (Nutrient Broth 8 g/l, yeast extract 5g/l and Dextrose 10 g/l) were inoculated with 100 ml of an overnight culture suspension of the strain having a known concentration (1 x 10⁵ CFU/ml). The flasks thus inoculated, were positioned on a rotary shaker at 150 rpm (revs per minute) and incubated at 23°C for 48 hours. Finally, the cultures were centrifuged and the pellet of the antagonist was resuspended in an adequate aliquot of sterile distilled water to obtain a sufficient concentration of the antagonist for applicative purposes.

### EXAMPLE 2: Isolation and fermentation of the strain of Cryptococcus laurentii coded DBVPG 31P.

*Cryptococcus laurentii* coded DBVPG 31P was isolated from the fruit of apple trees of cv Limongella, according to the technique described by Wilson et al., 1993, analogously to what is described in Example 1.

### EXAMPLE 3: Effectiveness tests of the strains Rhodosporidium kratochvilovae coded DBVPG 26P and Cryptococcus laurentii coded DBVPG 31P in the control of Blumeria (Erysiphae) graminis (powdery mildew) on durum wheat.

In order to obtain the culture suspension of the two strains DBVPG 26P of *Rhodosporidium kratochvilovae* and DBVPG 31P of *Cryptococcus laurentii,* 5 litre flasks containing the culture medium NYDB were inoculated with 100 ml of an overnight culture of a suspension of the strain having a known concentration (1x10⁵CFU/ml) previously prepared. The flasks thus inoculated were positioned on a rotary shaker at 150 rpm (revs per minute) and incubated at 23°C for 48 hours. Finally, the cultures were centrifuged in order to recover the antagonist cells which were resuspended in an adequate volume of sterile distilled water to obtain an antagonist concentration equal to 1.5-5 x 10⁶ CFU/ml.

The two microorganisms were tested on a culture of durum wheat against *Blumeria graminis* and were applied both individually and in combination, using as comparison (with the same concentrations) the strains LS11 and LS28 described in WO2008/114304.

The suspensions of the antagonists were applied on the leaf surface of wheat with a precision atomizer (operating pressure of 4 bar), spraying the equivalent of 400 l/ha. The experiments were performed according to a totally randomized block scheme wherein each thesis envisaged 5 replicates and each replicate was represented by plots of 100 m² (10 x 10 m). The activity results, expressed as the % reduction of durum wheat powdery mildew with respect to the non-treated reference (application of water alone), are reported in Table 1. This table indicates the activity of the two yeast strains used alone or combined with each other (DBVPG 26P + DBVPG 31P; LS11 + LS28).

In order to evaluate the synergistic effects, it has been used:
a) the Colby formula: Aₜ = A_{A} + A_{B} - A_{A}xA_{B}/100, wherein:
   Aₜ is the activity theoretically expected for the mixture of the two components A + B at a dosage D_{A} + D_{B};
   A_{A} is the activity detected for component A at the dosage D_{A};
   A_{B} is the activity detected for component B at the dosage D_{B};
b) the additive activity: A_{ad} = A_{A} + A_{B}.

If the activity detected for the mixture (A_{AB}) is higher than both the theoretical activity calculated according to Colby, and the additive activity, there is definitely a synergistic effect, which becomes stronger with an increase in the synergy factor SF = A_{AB}:Aₜ.

**Table 1. - Activity of the yeast strains DBVPG 26P, DBVPG 31P, LS11, LS28 and combinations thereof in the control of Blumeria graminis**

| **Yeast** | **Activity at dosages:** | **Theoretical activity** | **Additive activity** |
|---|---|---|---|
| | **5x10⁶ CFU/ml** | | |
| | **1. 5x10⁶ CFU/ml** | **Aₜ (SF)** | **A_{ad}** |
| **DBVPG 26P** | 80 | - | - |
| | 33 | - | - |
| **LS11** | 50 | - | - |
| | 15 | - | - |
| **DBVPG 31P** | 90 | - | - |
| | 40 | - | - |
| **LS28** | 65 | - | - |
| | 22 | - | - |
| **DBVPG 26P+ DBVPG 31P** | 90 | 59.8 (1.50) | 73 |
| **(1.5+1.5)x10⁶** | | | |
| **CFU/ml** | | | |
| **LS11+LS28** | 40 | 33.7 (1.18) | 37 |
| **(1.5+1.5)x10⁶** | | | |
| **CFU/ml** | | | |

In short, the results demonstrated the following:
- the strain DBVPG 26P of *Rhodosporidium kratochvilovae* is much more effective than the strain *Rhodotorula glutinis* coded LS11;
- the strain DBVPG 31P of *Cryptococcus laurentii* is more effective than the strain coded LS28;
- the combination of the two strains DBVPG 26P + DBVPG 31P, both at a dosage of 1.5x10⁶ CFU/ml, is more effective and has a synergistic effect significantly higher with respect to the combination LS11 + LS28 (described in WO2008/114304), both at a dosage of 1.5x10⁶ CFU/ml.

Furthermore, the results relating to the experiments effected for verifying the degradation rate of the mycotoxin patulin caused by the biological control agents, surprisingly revealed that the new isolated strain DBVPG 26P of *Rhodosporidium kratochvilovae* operate a mycotoxin degradation level equal to approximately the double with respect to what was observed for the isolated strain LS11 of *Rhodotorula glutinis* indicated in WO2008/114304.

## Claims

1. Yeast strain belonging to the species *Rhodosporidium kratochvilovae* having deposit number DBVPG 26P.

2. Yeast strain belonging to the species *Cryptococcus laurentii* having deposit number DBVPG 31P.

3. Mixture comprising or consisting of the yeast strains as defined according to claims 1 and 2.

4. Fungicidal composition **characterized in that** it comprises a yeast strain according to claim 1 or 2 or a mixture thereof as defined in claim 3 as active ingredient, together with other adjuvants and/or excipients phytopharmaceutically acceptable.

5. Fungicidal composition according to claim 4, wherein said strains when present in mixture are in a mutual ratio comprising between 4:1 and 1:4 in terms of Colony Forming Units (CFU).

6. Fungicidal composition according to anyone of the claims 4-5, wherein said strains are present at a concentration comprised between 5x10⁶ CFU/ml and 1x10⁸ CFU/ml, preferably comprised between 5x10⁷ CFU/ml and 1x10⁸ CFU/ml.

7. Composition according to any of the claims 4-6, **characterized in that** it comprises at least a further active principle selected between fungicide, phytoregulator, antibiotic, herbicide, insecticide, acaricide, fertilizer, alimentary additive, antioxidant and/or mixtures thereof.

8. Use of a yeast strain according to claim 1 or 2 or of the mixture according to claim 3, or the composition according to anyone of the claims 4-7 as a fungicide for controlling phytopathogenic fungi, also mycotoxigenic, in agricultural crops.

9. Use according to claim 8, wherein said phytopathogenic fungi, also mycotoxigenic, are selected from the group consisting of *Basidiomycetes, Ascomycetes, Deuteromycetes* or imperfect fungi, *Oomycetes* such as *Puccinia* spp., *Ustilago* spp., *Tilletia* spp., *Uromyces* spp., *Claviceps purpurea, Phakopsora* spp., *Rhizoctonia* spp., *Sclerotium rolfsii, Sclerotium cepivorum, Erysiphe* spp., *Sphaerotheca* spp., *Podosphaera* spp., *Uncinula* spp., *Helminthosporium* spp., *Rhynchosporium* spp., *Pyrenophora* spp., *Aspergillus* spp., *Botrytis* spp., *Monilinia* spp., *Sclerotinia* spp., *Rhizopus* spp., *Mucor* spp., *Septoria* spp. *(Mycosphaerella* spp.), *Phaeosphaeria* spp., *Venturia* spp., *Alternaria* spp., *Fusarium* spp., *Cercospora* spp., *Cladosporium* spp., *Phoma* spp., *Cercosporella herpotrichoides, Colletotrichum* spp., *Pyrenochaeta lycopersici, Pyricularia oryzae, Sclerotium* spp., *Ascochyta* spp., *Albugo* spp., *Phytophthora* spp., *Pythium* spp., *Plasmopara viticola, Thielaviopsis basicola, Diaporthe* spp, *Peronospora* spp., *Pseudoperonospora cubensis, Bremia lactucae, Penicillium* spp., *Cladosposium* spp., *Stemphylium* spp., *Leveillula taurica.*

10. Use according to anyone of the claims 8-9, wherein said agricultural crops are selected from the group consisting of cereals, fruits, citrus, leguminous, horticultural crops, cucurbitaceous, oleaginous plants, tobacco, coffee, tea, cacao, sugar beet, sugar cane, cotton.

11. Use according to anyone of the claims 8-10, **characterized in that** the strains are in the dried, freeze-dried, frozen, liquid state.

12. Use according to anyone of the claims 8-11, **characterized in that** the mixture or the composition is formulated in the form of dry powder, wettable powder, solution, dispersion or suspension.

13. Method for the control of phytopathogenic fungi in agricultural crops by the application pre- or post-harvest of an yeast strain as defined according to anyone of claim 1 or 2, or of a mixture thereof as defined according to claim 3, or a composition as defined according to anyone of the claims 4-7 as fungicide for the control of phytopathogenic fungi, also mycotoxigenic, in agricultural crops.

14. Method according to claim 13, **characterized in that** said application is carried out on a wounded or integral surface of the plant selected between fruits, leaves, flowers, stems, buds, branches and roots, on the seeds before seeding, or on the ground wherein the plant is growing.

15. Method according to anyone of the claims 13-14, wherein the dosage employed is comprised between 1 and 20 kg per hectare of composition containing the yeast strain or the yeast strains.

## Patentansprüche

1. Hefestamm, der zur Art *Rhodosporidium kratochvilovae* mit der Hinterlegungsnummer DBVPG 26P gehört.

2. Hefestamm, der zur Art *Cryptococcus laurentii* mit der Hinterlegungsnummer DBVPG 31P gehört.

3. Mischung, umfassend oder bestehend aus den Hefestämmen, wie sie in den Ansprüchen 1 und 2 definiert sind.

4. Fungizide Zusammensetzung, **dadurch gekennzeichnet, dass** sie einen Hefestamm nach Anspruch 1 oder 2 oder eine Mischung davon, wie in Anspruch 3 definiert, als Wirkstoff zusammen mit anderen phytopharmazeutisch verträglichen Hilfs- und/oder Trägerstoffen umfasst.

5. Fungizide Zusammensetzung nach Anspruch 4, wobei die Stämme, wenn in Mischung vorliegend, in einem gegenseitigen Verhältnis zwischen 4:1 und 1:4 bezogen auf die koloniebildenden Einheiten (KBE) stehen.

6. Fungizide Zusammensetzung nach einem der Ansprüche von 4 bis 5, wobei die Stämme in einer Konzentration zwischen 5x10⁶ KBE/ml und 1x10⁸ KBE/ml, vorzugsweise zwischen 5x10⁷ KBE/ml und 1x10⁸ KBE/ml, vorliegen.

7. Zusammensetzung nach einem der Ansprüche von 4 bis 6, **dadurch gekennzeichnet, dass** sie mindestens ein weiteres Wirkprinzip umfasst, das aus Fungizid, Phytoregulator, Antibiotikum, Herbizid, Insektizid, Akarizid, Düngemittel, Nahrungszusatzstoff, Antioxidationsmittel und/oder Gemischen davon ausgewählt ist.

8. Verwendung eines Hefestamms nach Anspruch 1 oder 2 oder der Mischung nach Anspruch 3 oder der Zusammensetzung nach einem der Ansprüche von 4 bis 7 als Fungizid zur Bekämpfung von phytopathogenen, auch Mykotoxin produzierenden, Pilzen bei landwirtschaftlichen Kulturpflanzen.

9. Verwendung nach Anspruch 8, wobei die phytopathogenen, auch Mykotoxin produzierenden, Pilze aus der Gruppe ausgewählt sind, die aus *Basidiomycetes, Ascomycetes*, *Deuteromycetes* oder Fungi imperfecti, *Oomycetes* wie *Puccinia* spp., *Ustilago* spp., *Tilletia* spp., *Uromyces* spp., *Claviceps purpurea, Phakopsora* spp., *Rhizoctonia* spp., *Sclerotium rolfsii, Sclerotium cepivorum, Erysiphe* spp., *Sphaerotheca* spp., *Podosphaera* spp., *Uncinula* spp., *Helminthosporium* spp., *Rhynchosporium* spp., *Pyrenophora* spp., *Aspergillus* spp., *Botrytis* spp., *Monilinia* spp., *Sclerotinia* spp., *Rhizopus* spp., *Mucor* spp., *Septoria* spp. (*Mycosphaerella* spp.), *Phaeosphaeria* spp., *Venturia* spp., *Alternaria* spp., *Fusarium* spp., *Cercospora* spp., *Cladosporium* spp., *Phoma* spp., *Cercosporella herpotrichoides*, *Colletotrichum* spp., *Pyrenochaeta lycopersici*, *Pyricularia oryzae*, *Sclerotium* spp., *Ascochyta* spp., *Albugo* spp., *Phytophthora* spp., *Pythium* spp., *Plasmopara viticola, Thielaviopsis basicola*, *Diaporthe* spp, *Peronospora* spp., *Pseudoperonospora cubensis*, *Bremia lactucae*, *Penicillium* spp., *Cladosposium* spp., *Stemphylium* spp. und *Leveillula taurica* besteht.

10. Verwendung nach einem der Ansprüche von 8 bis 9, wobei die landwirtschaftlichen Kulturpflanzen aus der Gruppe ausgewählt sind, die aus Getreide, Obst, Zitrusgewächsen, Hülsenfrüchten, gartenbaulichen Kulturpflanzen, Kürbisgewächsen, Ölpflanzen, Tabak, Kaffee, Tee, Kakao, Zuckerrübe, Zuckerrohr und Baumwolle besteht.

11. Verwendung nach einem der Ansprüche von 8 bis 10, **dadurch gekennzeichnet, dass** sich die Stämme im getrockneten, gefriergetrockneten, gefrorenen, flüssigen Zustand befinden.

12. Verwendung nach einem der Ansprüche von 8 bis 11, **dadurch gekennzeichnet, dass** die Mischung oder die Zusammensetzung in Form von Trockenpulver, benetzbarem Pulver, Lösung, Dispersion oder Suspension formuliert ist.

13. Verfahren zur Bekämpfung von phytopathogenen Pilzen bei landwirtschaftlichen Kulturpflanzen durch die Anwendung vor oder nach der Ernte eines Hefestamms, wie er in einem der Ansprüche 1 oder 2 definiert ist, oder einer Mischung davon, wie sie in Anspruch 3 definiert ist, oder einer Zusammensetzung, wie sie in einem der Ansprüche von 4 bis 7 definiert ist, als Fungizid zur Bekämpfung von phytopathogenen, auch Mykotoxin produzierenden, Pilzen bei landwirtschaftlichen Kulturpflanzen.

14. Verfahren nach Anspruch 13, **dadurch gekennzeichnet, dass** die Anwendung auf einer verwundeten oder unversehrten Pflanzenoberfläche, die unter Früchten, Blättern, Blüten, Stielen, Knospen, Zweigen und Wurzeln ausgewählt wird, oder auf den Samen vor der Aussaat oder auf dem Boden, in dem die Pflanze wächst, durchgeführt wird.

15. Verfahren nach einem der Ansprüche von 13 bis 14, wobei die verwendete Dosierung zwischen 1 und 20 kg pro Hektar der Zusammensetzung beträgt, die den Hefestamm oder die Hefestämme enthält.

## Revendications

1. Souche de levure appartenant à l'espèce Rhodosporidium kratochvilovae ayant le numéro de dépôt DBVPG 26P.

2. Souche de levure appartenant à l'espèce Cryptococcus laurentii ayant le numéro de dépôt DBVPG 31P.

3. Mélange comprenant ou consistant en les souches de levure telles que définies selon les revendications 1 et 2.

4. Composition fongicide **caractérisée en ce qu'**elle comprend une souche de levure selon la revendication 1 ou 2 ou un mélange de celles-ci telle que définie dans la revendication 3 comme substance active, conjointement à d'autres adjuvants et/ou excipients phytopharmaceutiquement acceptables.

5. Composition fongicide selon la revendication 4, dans laquelle lesdites souches, quand elles sont présentes en mélange, sont dans un rapport mutuel compris entre 4:1 et 1:4 en termes d'unités formant colonies (CFU).

6. Composition fongicide selon l'une quelconque des revendications 4 à 5, dans laquelle lesdites souches sont présentes à une concentration comprise entre 5x10⁶ CFU/ml et 1x10⁸ CFU/ml, de préférence comprise entre 5x10⁷ CFU/ml et 1x10⁸ CFU/ml.

7. Composition selon l'une quelconque des revendications 4 à 6, **caractérisée en ce qu'**elle comprend au moins un autre principe actif sélectionné parmi fongicide, phytorégulateur, antibiotique, herbicide, insecticide, acaricide, engrais, additif alimentaire, antioxydant et/ou des mélanges de ceux-ci.

8. Utilisation d'une souche de levure selon la revendication 1 ou 2 ou du mélange selon la revendication 3 ou de la composition selon l'une quelconque des revendications 4 à 7 comme un fongicide pour combattre des champignons phytopathogènes, également mycotoxigènes, sur des cultures agricoles.

9. Utilisation selon la revendication 8, dans laquelle lesdits champignons pathogènes, également mycotoxigènes, sont sélectionnés dans le groupe comprenant *Basidiomycètes, Ascomycètes, Deuteromycètes* ou champignons de type imperfecti, *Oomycètes* tels que *Puccinia* spp., *Ustilago* spp., *Tilletia* spp., *Uromyces* spp., *Clavicepts pupurea, Phakospora* spp., *Rhizoctonia* spp., *Sclerotium rolfsii, Sclerotium cepivorum, Eryphe* spp., *Sphaerotheca* spp., *Podosphaera* spp., *Uncinula* spp., *Helminthosporium* spp., *Rhynchosporium* spp., *Pyrenophora* spp., *Aspergillus* spp., *Botrytis* spp., *Monilinia* spp., *Sclerotinia* spp., *Rhizopus* spp., *Mucor* spp., *Septoria* spp. *(Mycosphaerella* spp.), *Phaeosphaeria* spp., *Venturia* spp., *Alternaria* spp., *Fusarium* spp., *Cercospora* spp., *Cladosporium* spp., *Phoma* spp., *Cercosporella herpotrichoïdes, Colletotrichum* spp., *Pyrenochaeta lycopersici, Pyricularia oryzae, Sclerotium* spp., *Ascochyta* spp., *Albugo* spp, *Phytophthora* spp., *Pythium* spp., *Plasmopara viticole, Thielaviopsis basicola, Diaporthe* spp., *Peronospora* spp., *Pseudoperonospora cubensis, Bremia lactucae, Penicillium* spp., *Cladosporium* spp., *Stemphylium* spp., *Leveillula taurica.*

10. Utilisation selon l'une quelconque des revendications 8 à 9, dans laquelle lesdites cultures agricoles sont sélectionnées dans le groupe comprenant les céréales, les fruits, les agrumes, les légumineuses, les cultures horticoles, les cucurbitacées, les plantes oléagineuses, le tabac, le café, le thé, le cacao, la betterave à sucre, la canne à sucre, le coton.

11. Utilisation selon l'une quelconque des revendications 8 à 10, **caractérisée en ce que** les souches sont dans un état liquide, desséché, lyophilisé, congelé.

12. Utilisation selon l'une quelconque des revendications 8 à 11, **caractérisée en ce que** le mélange ou la composition est formulée sous la forme de poudre sèche, poudre mouillable, solution, dispersion ou suspension.

13. Procédé pour combattre des champignons phytopathogènes sur des cultures agricoles par l'application pré- ou post-récolte d'une souche de levure selon la revendication 1 ou 2 ou d'un mélange selon la revendication 3 ou d'une composition selon l'une quelconque des revendications 4 à 7 en tant que fongicide pour combattre des champignons phytopathogènes, également mycotoxigènes, sur des cultures agricoles.

14. Procédé selon la revendication 13, **caractérisé en ce que** ladite application est exécutée sur une surface lésée ou intacte de la plante sélectionnée parmi les fruits, les feuilles, les fleurs, les tiges, les bougeons, les branches et les racines, sur les semences avant l'ensemencement ou sur le terrain dans lequel la plante est cultivée.

15. Procédé selon l'une quelconque des revendications 13 à 14, dans lequel le dosage employé est compris entre 1 et 20 kg par hectare de composition contenant la souche de levure ou les souches de levure.
